# EUROPEAN PATENT SPECIFICATION

(11) **EP 2 352 750 B1**
(45) Date of publication and mention of the grant of the patent: **13.06.2012**
(21) Application number: 10755307.5
(22) Date of filing: 17.09.2010
(51) Int. Cl.: C07K 14/435

(54) **BUFFERS FOR CONTROLLING THE PH OF BONE MORPHOGENETIC PROTEINS**
PUFFER ZUR STEUERUNG DES PH-WERTES KNOCHENMORPHOGENETISCHER PROTEINE
TAMPONS UTILISÉS POUR LA RÉGULATION DU PH DES PROTÉINES MORPHOGÉNÉTIQUES OSSEUSES

(30) Priority: 17.09.2009 US 243383 P
(43) Date of publication of application: 10.08.2011
(73) Proprietor: Stryker Corporation, Kalamazoo, MI 49002 (US)
(72) Inventor: HILE, David, Westborough MA 01581 (US); STROHMEIER, Gregg, Newton MA 02450 (US)
(74) Representative: Bridle, Andrew Barry
(86) International application number: PCT/US2010/049229
(87) International publication number: WO 2011/035094

(56) References cited:
- EP-A1- 1 374 905
- EP-A2- 1 348 450
- WO-A1-00/23123
- WO-A1-01/78687
- MURAKAMI NARUMICHI ET AL: "Repair of segmental defects in rabbit humeri with titanium fiber mesh cylinders containing recombinant human bone morphogenetic protein-2 (rhBMP-2) and a synthetic polymer", JOURNAL OF BIOMEDICAL MATERIALS RESEARCH, vol. 62, no. 2, November 2002 (2002-11), pages 169-174, XP9141410, ISSN: 0021-9304
- SCHWENDEMAN S: "recent advances in the stabilization of proteins encapsulated in injectable PLGA delivery systems", CRITICAL REVIEWS IN THERAPEUTIC DRUG CARRIER SYSTEMS, BEGELL HOUSE PUBLISHING INC, US, vol. 19, 1 January 2002 (2002-01-01), pages 73-98, XP008108131, ISSN: 0743-4863

## Description

### Cross-Reference to Related Applications

This application claims priority to and the benefit of U.S. Provisional Patent Application Number 61/243,383, filed September 17, 2009, the contents of which are incorporated by reference herein.

### Technical Field of the Invention

The present invention is related to compositions for stabilizing the pH of bone morphogenetic protein compositions.

### Background

The stability of solutions and lyophilized formulations of cysteine-knot proteins and TGF-β superfamily proteins, which includes the family of bone morphogenetic proteins (BMPs), depends on the pH of the formulation. These proteins are basic and, when added to a neutral buffer, the pH increases. Addition of low concentrations of these proteins does not significantly affect the resulting pH of the formulation. However, as the concentration of these proteins in a formulation increases, the pH of the formulation increases, thereby lowering the stability of these formulations. Accordingly, to return the formulation to a desired pH, a strong acid, such as HCl, is added. Strong acids are used to reduce the volume required to return the pH within the desired range. However, adding HCl increases the ionic strength of the formulations, thereby decreasing their stability. Accordingly, there is a need to identify new methods and compositions that better control pH of cysteine-knot proteins and TGF-β superfamily proteins, including BMPs. Murakami Narumichi et al. (2002) Journal of Biomedical Materials Research 62(2): 169-174 discloses rhBMP-2 as a solution in a buffer containing 2.5% glycine. WO01/78687, EP1374905, EP1348450 and WO0023123 disclose the use of acidifying agents in the preparation of pharmaceutical compositions comprising various bone morphogenic proteins. However, none of these documents discloses buffer solutions comprising bone morphogenic proteins and glycylglycine or tartaric acid buffers.

### Summary of the Invention

The present invention is based on the discovery that certain buffers enhance the stability of formulations of cysteine-knot proteins and TGF-β superfamily proteins, which includes the family of bone morphogenetic proteins (BMPs), particularly liquid or solution formulations and lyophilized formulations. In particular, when these proteins, which are basic, are added to certain buffers in high concentrations, the pH of the buffer increases. A limiting factor to date for optimal use of BMPs, particularly in therapeutic regimens, has been that liquid solution BMP formulations having pHs greater than 3.0 ± 0.2 and lyophilized BMP formulations having pH greater than 3.5 are not stable long-term and are subject to denaturation and aggregation. Use of prior art buffers such as sodium lactate has limited the BMP concentrations in formulations and has also limited the stability of BMPs in these formulations as strong acids must be added to stabilize the pH, thereby increasing the ionic strength of the formulations. The present invention allows the skilled artisan to prepare a more highly concentrated, stable BMP formulation for use in various therapeutic indications. Further, the invention provides a more robust manufacturing process that permits greater control of pH during the manufacturing process thereby providing greater uniformity between lots of BMP formulations produced.

In one aspect, the invention contemplates a solution including a bone morphogenetic protein and an aqueous glycylglycine buffer. According to one embodiment, the bone morphogenetic protein can be any of BMP-2, BMP-4, BMP-5, BMP-6, BMP-7, GDF-5, GDF-6, or GDF-7. In a preferred embodiment, the BMP is BMP-7. The pH of the solution can range from about 2.5 to about 4.0, more preferably from about 2.8 to about 3.5, and even more preferably from about 2.8 to about 3.2. The glycylglycine buffer can have a concentration from about 1 mM to about 100 mM, from about 2 mM to about 20 mM, even more from about 5 mM to about 15 mM, or about 10 mM. The concentration of the bone morphogenetic protein in the solution can be from about 0.01 mg/mL to about 40 mg/mL, from about 0.1 mg/mL to about I mg/mL, or from about 0.1 mg/mL to about 20 mg/mL, or from about 1 mg/mL to about 20 mg/mL. According to one embodiment, the pH of the solution does not vary more than about 0.2 pH units upon storage at 5-40°C for six months, or as long as 36 months.

The solution can also include a lyoprotectant, for example, sugar, such as mannitol, mannose, lactose, sucrose, or trehalose, or any combinations of these sugars. In another embodiment, the lyoprotectant is preferably mannitol, mannose, or trehalose. For example, trehalose can be included in the amount of about 1% to about 15% (w/v), or from about 3% to about 10% (w/v). In a preferred embodiment, trehalose does not exceed 9% (w/v).

The solution can also include an antioxidant. For example, the antioxidant can be methionine, ascorbic acid, benzyl alcohol, glutathione, m-cresol, EDTA, sodium metabisulfate, or thioglycerol. In a preferred embodiment, the antioxidant is methionine. In yet another embodiment, the solution does not contain an antioxidant.

In another aspect, the invention contemplates a solid composition including a bone morphogenetic protein and glycylglycine, a glycylglycine salt, or a combination of glycylglycine and a glycylglycine salt. In a further embodiment, the glycylglycine salt is glycylglycine HCl. The solid composition can include a lyoprotectant, for example, a sugar. The sugar can be mannitol, lactose, sucrose, or trehalose or any combination of these sugars. For example, the sugar can be trehalose which can be included, for example, at a ratio of bone morphogenetic protein to trehalose of about 7x10⁻⁵:1 (w/w) to about 1.3:1 (w/w). The ratio of bone morphogenetic protein to glycylglycine, glycylglycine salt, glycylglycine HCl or combination of glycylglycine and glycylglycine salt can be, for example, about 8x10^{-4:}:1 (w/w) to about 300:1 (w/w). The solid composition can also include an antioxidant. The antioxidant can be, for example, ascorbic acid, benzyl alcohol, glutathione, m-cresol, thioglycerol, and methionine. In a preferred embodiment, the antioxidant is methionine.

The solid composition can also include a surfactant. For example, in one embodiment, the surfactant is one of polysorbate 20, polysorbate 80, or poloxamer 407. The surfactant can be present in a range of about 0.001% to 0.5% (w/w). In a preferred embodiment, the surfactant is polysorbate 20 at about 0.01% (w/w). In one embodiment, the composition does not include a surfactant.

A solid composition can be prepared by lyophilizing the solution of bone morphogenetic protein and glycylglycine. In one embodiment, the lyophilized composition does not include mannitol.

In yet another aspect, the invention contemplates a solution including a bone morphogenetic protein and an aqueous tartaric acid buffer. In one embodiment, the bone morphogenetic protein can be any of BMP-2, BMP-4, BMP-5, BMP-6, BMP-7, GDF-5, GDF-6, or GDF-7. In a preferred embodiment, the BMP is BMP-7. The pH of the solution can range from about 2.5 to about 4.0, more preferably from about 2.8 to about 3.5, and even more preferably from about 2.8 to about 3.2. The tartaric acid buffer can have a concentration from about 1 mM to about 100 mM, from about 2 mM to about 20 mM, even more from about 5 mM to about 15 mM, or about 10 mM. The concentration of the bone morphogenetic protein in the solution is from about 0.01 mg/mL to about 40 mg/mL, from about 1 mg/mL to about 20 mg/mL, from about 0.1 mg/mL to about 1 mg/mL, or from about 0.1 mg/mL to about 20 mg/mL. In one embodiment, the pH of the solution does not vary more than about 0.2 pH units in a liquid formulation, as a lyophilizate, or as a reconstituted solution prepared from a lyophilizate.

In the case of the tartaric acid invention, the solution can also include a lyoprotectant, for example, sugar, such as mannitol, lactose, sucrose, or trehalose, or any combinations of these sugars. For example, trehalose can be included in the amount of about 1% to about 15% (w/v), or from about 3% to about 10% (w/v). In a preferred embodiment, trehalose does not exceed 9% (w/v). The solution can also include an antioxidant. For example, the antioxidant can be methionine, ascorbic acid, benzyl alcohol, glutathione, m-cresol, or thioglycerol. In a preferred embodiment, the antioxidant is methionine. In yet another embodiment, the solution does not contain an antioxidant. The solid composition can also include a surfactant. For example, in one embodiment, the surfactant is polysorbate 20, polysorbate 80, or poloxamer 407. The surfactant, is present, for example, in a range of about 0.001% to 0.5% (w/w). In a preferred embodiment, the surfactant is polysorbate 20 at about 0.01% (w/w). In one embodiment, the composition does not include a surfactant.

In a related aspect, the invention contemplates a solid composition including a bone morphogenetic protein and tartaric acid, a tartrate salt, or a combination of tartaric acid and a tartrate salt. The tartaric acid and/or tartrate salt can include any enantiomers or stereoisomers of tartrate including (+)tartaric acid (also known as dextrotartaric acid), (-)tartaric acid (also known as levotartaric acid), combinations thereof (also known as racemic acid), and stereoisomers of dextrotartaric acid (also known as mesotartaric acid). In a preferred embodiment, the tartaric acid is (+)tartaric acid or dextrotartaric acid. The solid composition can include a lyoprotectant, for example, a sugar. The sugar can be mannitol, lactose, sucrose, or trehalose or any combination of these sugars. For example, the sugar can be trehalose which can be included, for example, at a ratio of bone morphogenetic protein to trehalose of about 7x 10⁻⁵:1 (w/w) to about 1.3:1 (w/w). The ratio of bone morphogenetic protein to tartaric acid, tartrate salt, or combination of tartaric acid and tartrate salt can be, for example, about 7x10⁻⁴:1 to about 270:1 (w/w). In a preferred embodiment, the ratio is about 0.07:1 to about 13:1 (w/w) protein to tartaric acid, tartrate salt, or combination of tartaric acid and tartrate salt. The solid composition can also include an antioxidant. The antioxidant can be, for example, ascorbic acid, benzyl alcohol, glutathione, m-cresol, thioglycerol, and methionine. The solid composition can also include a surfactant such as polysorbate 20, polysorbate 80, poloxamer 188, or poloxamer 407. The surfactant can be present in a range of about 0.001 % to 0.5% (w/w). In a preferred embodiment, the surfactant is polysorbate 20 at about 0.01% (w/w). In another embodiment, the composition does not contain a surfactant.

A solid composition can be prepared by lyophilizing the solution of bone morphogenetic protein and tartaric acid. In one embodiment, the lyophilized composition does not include mannitol.

### Brief Description of the Figures

FIG. 1 is a line graph plotting the concentration of BMP-7 (mg/mL) versus the pH of a solution containing either tartaric acid (1), glycylglycine (2), malic acid (3), lactic acid (4), aspartic acid (5), or succinic acid (6) and 9% trehalose as described in Example 2.

FIG. 2 is a line graph plotting the percent aggregation of BMP-7 over time in the presence of glycylglycine pH 3.1 (1), lactate pH 3.5 (2), tartrate pH 3.1 (3), lactate plus 10 mM NaCl pH 3.1 (4), and lactate plus 10 mM NaCl, 20 mM methionine pH 3.1 (5) as described in Example 3.

FIG. 3A is a line graph plotting the percent aggregation over time of a lyophilized formulation of 1 mg/mL BMP-7 held at 40°C, while FIG. 3B is a line graph plotting the percent aggregation over time of a lyophilized formulation of 16 mg/mL, BMP-7 held at 40°C.

FIG. 4 is a line graph plotting the percent aggregation over time of a lyophilized formulation of 1 mg/mL BMP-7 containing 10 mM glycylglycine, 10 mM lactate or 10 mM tartrate.

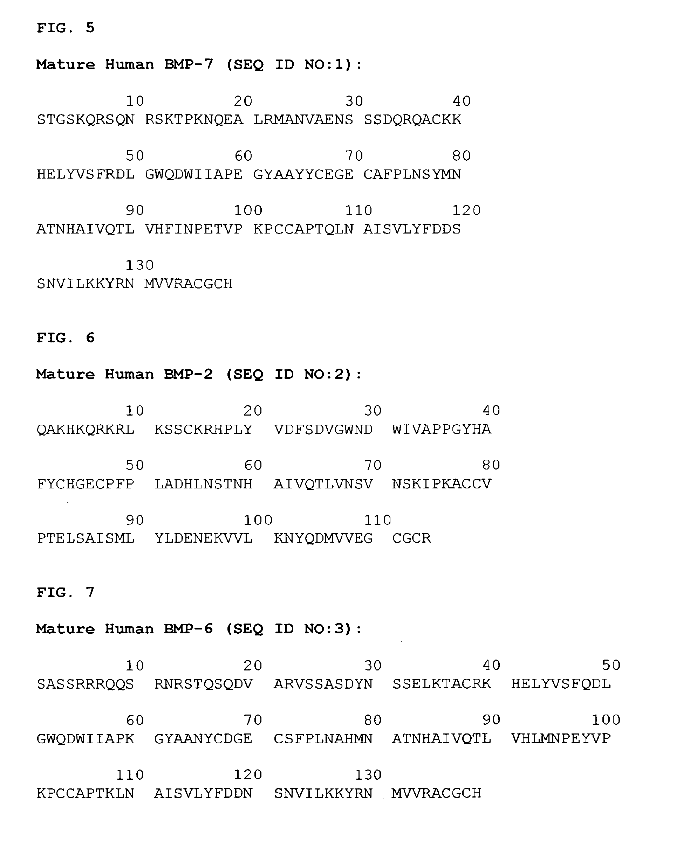

FIG. 5 is the amino acid sequence of mature human BMP-7 (SEQ ID NO:1).

FIG. 6 is the amino acid sequence of mature human BMP-2 (SEQ ID NO:2).

FIG. 7 is the amino acid sequence of mature human BMP-6 (SEQ ID NO:3).

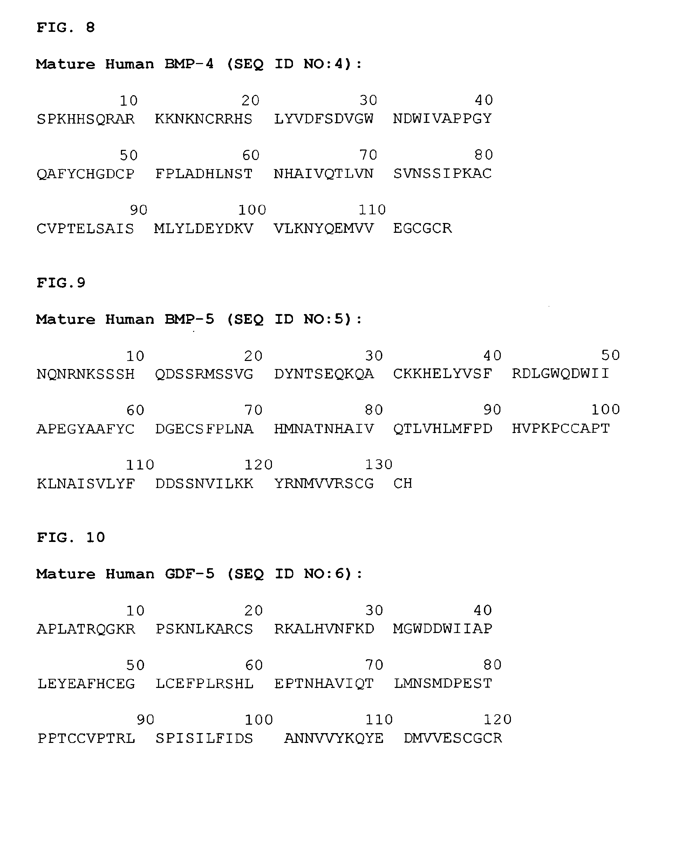

FIG. 8 is the amino acid sequence of mature human BMP-4 (SEQ ID NO:4).

FIG. 9 is the amino acid sequence of mature human BMP-5 (SEQ ID NO:5).

FIG. 10 is the amino acid sequence of mature human GDF-5 (SEQ ID NO:6).

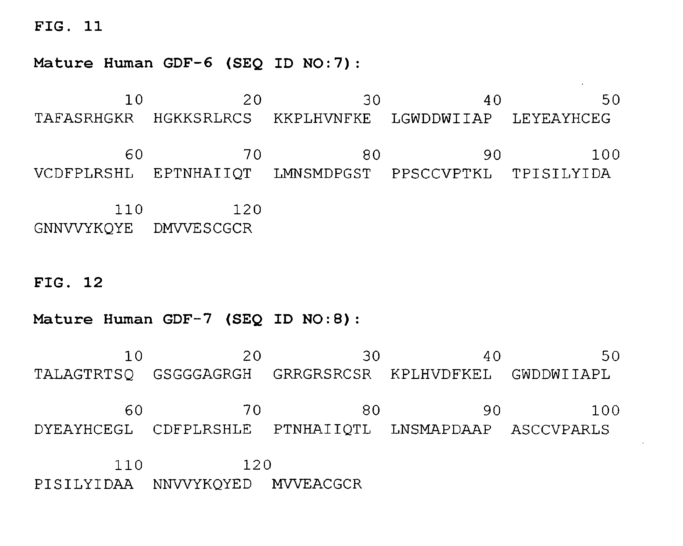

FIG. 11 is the amino acid sequence of mature human GDF-6 (SEQ ID NO:7).

FIG. 12 is the amino acid sequence of mature human GDF-7 (SEQ ID NO:8).

### Detailed Description of the Invention

The stability of liquid or reconstituted cysteine-knot family proteins including TGF-β superfamily proteins such as BMPs is dependent upon the pH and the ionic strength of the formulation, with the desired pH range being 3.0±0.2. Adding low concentrations of these proteins, *i.e.,* less than 2 mg/mL, does not significantly affect the pH of the resulting formulation. However, as the concentration of these proteins increases, *i.e.,* greater than 2 mg/mL, the pH of the formulation increases, *e.g.,* pH greater than 3.4. Because of the instability of these proteins at increased ionic strength, there is a limit to the concentration of buffer and pH-adjusting acids that can be used to mitigate the observed increase in pH.

Applicants have made the surprising and unexpected discovery that preparing these protein formulations, such as with BMPs, using an aqueous glycylglycine buffer or tartaric acid buffer eliminates the need to reduce the pH of the formulation by adding a strong acid after the protein is added.

The advantages of selecting a buffer that controls pH throughout a wider BMP concentration range include fewer manufacturing process manipulations, including eliminating or reducing the need to adjust the pH of the formulation with a strong acid, *e.g.,* HCl, or strong base, *e.g.,* NaOH. This enhances the stability of the BMP drug product by reducing the ionic strength of the formulation, reduces denaturation and/or aggregation of the BMP protein, and increases the feasible concentration range for a stable, lyophilized drug product of BMP.

It is important that formulations containing BMPs are prepared at the appropriate pH 3.0 ±0.2 as members of the BMP family of proteins are inherently insoluble under physiological conditions, *i.e.,* at physiologic pH, especially at concentrations in excess of about 1 mg/mL. Accordingly, it is necessary to control the pH to ensure solubility of the necessary quantities of BMP protein in the formulation.

### Formulations

Formulations according to the invention include solutions (liquid form) such as, but not limited to reconstituted lyophilizates and solid forms such as, but not limited to lyophilized forms, gels, microencapsulated particles, and pastes. Combinations of liquid formulations, lyophilizates, and liquid solutions prepared from reconstituted lyophilizates used in combination with gels, pastes, or particles are also contemplated by the invention.

For example, in one embodiment according to the invention, the formulation is a solution including an aqueous buffer and a BMP protein. For example, the buffer can be glycylglycine, tartaric acid, malic acid, lactic acid, aspartic acid, or succinic acid. In a preferred embodiment, the buffer is glycylglycine, while in another preferred embodiment, the buffer is tartaric acid. In another embodiment, the buffer is combination of two or more of glycylglycine, tartaric acid, malic acid, lactic acid, aspartic acid, or succinic acid. For example, in one embodiment, the buffer is glycylglycine and tartaric acid.

In yet another embodiment, the glycylglycine buffer has a concentration from about 1 mM to about 100 mM, more preferably from about 2 mM to about 20 mM, and even more preferably from about 5 mM to about 15 mM, and yet more preferably, the concentration of glycylglycine buffer is about 10 mM.

According to another embodiment, the liquid solution formulation further includes a stabilizer. For example, the stabilizer can be proline, glycine, valine, isoleucine, or leucine. In one preferred embodiment, the stabilizer is proline, while in yet another embodiment, the stabilizer is a combination of two or more of proline, glycine, valine, isoleucine, or leucine.

According to one embodiment, the BMP is BMP-7. According to another embodiment, the BMP is BMP-2, -4, -5, -6, or -9. According to another embodiment, the BMP is GDF-5, -6, or -7. The concentration of BMP in the formulation is, for example, about 0.01 mg/mL to about 40 mg/mL. In yet another embodiment, the concentration of BMP protein is about 1 mg/mL to about 20 mg/mL. A preferred concentration of BMP is about 0.1 mg/mL to about 1 mg/mL; a more preferred concentration of BMP is about 0.1 mg/mL to about 20 mg/mL; and a most preferred concentration of BMP is about 0.1 mg/mL to about 1 mg/mL.

In yet another embodiment, the pH of the formulation solution is from about 2.5 to about 4.0, more preferably from about 2.8 to about 3.5, and even more preferably about 2.8 to about 3.2.

In another embodiment, lyophilization of a liquid formulation of the invention disclosed herein yields a solid formulation.

In another embodiment, the formulation is a solid form derived from a solution of a buffer and a BMP protein. For example, the buffer can be glycylglycine, tartrate, malate, lactate, aspartate, succinate or a salt of glycylglycine, tartrate, malate, lactate, aspartate, or succinate. In a preferred embodiment, the buffer is glycylglycine or a salt of glycylglycine. In yet another preferred embodiment, the buffer is a tartrate salt.

According to another embodiment, the solid formulation includes a stabilizer. For example, the stabilizer can be proline, glycine, valine, isoleucine, or leucine. In one preferred embodiment, the stabilizer is proline. In yet another embodiment, the stabilizer is a combination of two or more of proline, glycine, valine, isoleuncine, or leucine.

According to one embodiment, the BMP is BMP-7. According to another embodiment, the BMP is BMP-2, -4, -5, -6, or -9. According to another embodiment, the BMP is GDF-5, -6, or -7.

In one embodiment, the ratio of BMP to the buffer is about 7.7x10⁻⁴:1 to about 310:1 (w/w). For example, the ratio of BMP to a glycylglycine salt is about 7.7x10⁻⁴:1 to about 310:1 (w/w). For example, the ratio of BMP to a tartaric acid or salt is about 6.7x10⁻⁴:1 to about 270: 1

In another embodiment, the liquid or solid form formulation further includes a lyoprotectant. In one embodiment, the lyoprotectant is a sugar. For example, the sugar can be mannitol, lactose, sucrose, or trehalose, or a combination of two or more of these sugars. In a preferred embodiment, the sugar is trehalose. The sugar can be added in an amount from about 1% to about 15% (w/v) to a formulation of the invention in liquid form, more preferably about 3% to about 10% (w/v). In a preferred embodiment, the sugar does not exceed 9% (w/v). In another embodiment, the sugar is present in an amount of about 1:7x10⁻⁵ of about 1:1.3 (w/w) sugar:BMP in a formulation in solid form.

In yet another embodiment, the liquid or solid form formulation further includes an antioxidant. For example, the antioxidant can be methionine, ascorbic acid, benzyl alcohol, glutathione, m-cresol, or thioglycerol. A preferred antioxidant is methionine.

Table 1 below shows the components of various glycylglycine buffers that can be made in accordance with the invention. Glycylglycine buffer consists of glycylglycine, glycylglycine-HCl, glycylglycine salts, or a combination thereof. Formulation components, specifically the percentage of trehalose, can be adjusted to yield the target osmolarity.

**Table 1. Glycylglycine Formulations**

| **Component** | **Range** | **Preferred** | **Ratio BMP: Component (Low)** | **Ratio BMP: Component (High)** |
|---|---|---|---|---|
| BMP | 0.01 - 40 mg/mL | 0.1 - 1 mg/mL or 1 - 20 mg/mL | N/A | N/A |
| Glycylglycine* | 1 - 100 mM (0.13 - 13 mg/mL) | 10 mM (1.3 mg/mL) | 7.7×10⁻⁴ (mg/mg) | 308 (mg/mg) |
| Trehalose | 3 - 14% (w/v) (30 - 140 mg/mL) | 9% (w/v) (90 mg/mL) | 7.1×10⁻⁵ (mg/mg) | 1.3 (mg/mg) |
| Methionine | 0 - 100 mM (0 - 15 mg/mL) | 20 mM, 3 mg/mL | 6.7×10⁻⁴ (mg/mg) | N/A (value would be 40/0 mg/mg) |
| Polysorbate 20 | 0 - 0.5% (w/v) (0 - 5 mg/mL) | 0.01% (w/v)) | 0.02 (mg/mg) | N/A (value would be 40/0 mg/mg) |
| Osmolality* | N/A | 300 ± 30 mOsm/kg | N/A | N/A |

Table 2 below shows the components of various tartrate buffers that can be made in accordance with the invention. Formulation components, specifically percentage of trehalose, can be adjusted to yield target osmolality.

**Table 2. Tartrate Formulations**

| **Component** | **Range** | **Preferred** | **Ratio BMP: Component (Low)** | **Ratio BMP: Component (High)** |
|---|---|---|---|---|
| BMP | 0.01 - 40 mg/mL | 0.1 - 1 mg/mL or 1 - 20 mg/mL | N/A | N/A |
| Tartaric acid* | 1 - 100mM (0.15 - 15 mg/mL) | 10 mM (1.5 mg/mL) | 6.7×10⁻⁴ (mg/mg) | 267 (mg/mg) |
| Trehalose | 3 - 14% (w/v) (30 - 140 mg/mL) | 9% (w/v) (90 mg/mL) | 7.1×10⁻⁵ (mg/mg) | 1.3 (mg/mg) |
| Methionine | 0 - 100 mM (0 - 15 mg/mL | 20 mM, 3 mg/mL | 6.7×10⁻⁴ (mg/mg) | N/A |
| Polysorbate 20 | 0 - 0.5% (w/v) (0 - 5 mg/mL) | 0.01 % (w/v) | 0.02 (mg/mg) | N/A |
| Osmolality** | N/A | 300 ± 30 mOsm/kg | N/A | N/A |

One of the unexpected benefits of the formulations of the invention is that the formulations maintain their stability over time. For example, when a solution formulation according to the invention is stored at 40°C for 6 months, the pH does not vary more than about 0.2 pH units.

### Bone Morphogenetic Proteins

BMPs are preferred exemplary proteins for the compositions of the present invention. BMPs belong to the TGF-β superfamily. The TGF-β superfamily proteins are cytokines characterized by six-conserved cysteine residues. The human genome contains about 42 open reading frames encoding TGF-β superfamily proteins. The TGF-β superfamily proteins can at least be divided into the BMP subfamily and the TGF-β subfamily based on sequence similarity and the specific signaling pathways that they activate. The BMP subfamily includes, but is not limited to, BMP-2, BMP-3 (osteogenin), BMP-3b (GDF-10), BMP-4 (BMP-2b), BMP-5, BMP-6, BMP-7 (osteogenic protein-1 or OP-1), BMP-8 (OP-2), BMP-8B (OP-3), BMP-9 (GDF-2), BMP-10, BMP-11 (GDF-11), BMP-12 (GDF-7), BMP-13 (GDF-6, CDMP-2), BMP-15 (GDF-9), BMP-16, GDF-1, GDF-3, GDF-5 (CDMP-1, MP-52), and GDF-8 (myostatin). For purposes of the present invention, preferred superfamily proteins include BMP-2, -4, -5, -6 and -7 and GDF-5, -6, and -7, as well as MP-52. Particularly preferred proteins include BMP-2, BMP-7 and GDF-5, -6, and -7. A most preferred exemplary BMP is human BMP-7. Furthermore, there is allelic variation in BMP sequences among different members of the human population, and there is species variation among BMPs discovered and characterized to date. As used herein, "BMP subfamily," "BMPs," "BMP ligands" and grammatical equivalents thereof refer to the BMP subfamily members, unless specifically indicated otherwise. Any of the members of the BMP subfamily disclosed herein can be included in formulations according to the invention.

The TGF-β subfamily includes, but is not limited to, TGFs (e.g., TGF-β1, TGF-β2, and TGF-β3), activins (e.g., activin A) and inhibins, macrophage inhibitory cytokine-1 (MIC-1), Mullerian inhibiting substance, anti-Mullerian hormone, and glial cell line derived neurotrophic factor (GDNF). As used herein, "TGF-β subfamily," "TGF-βs," "TGF-β ligands" and grammatical equivalents thereof refer to the TGF-β subfamily members, unless specifically indicated otherwise.

The TGF-β superfamily is in turn a subset of the cysteine knot Cytokine superfamily. Additional members of the cysteine knot cytokine superfamily include, but are not limited to, platelet derived growth factor (PDGF), vascular endothelial growth factor (VEGF), placenta growth factor (PIGF), noggin, neurotrophins (BDNF, NT3, NT4, and βNGF), gonadotropin, follitropin, lutropin, interleukin-17, and coagulogen.

Publications disclosing these sequences, as well as their chemical and physical properties, include: BMP-7 and OP-2 (U.S. Pat. No. 5,011,691; U.S. Pat. No. 5,266,683; Ozkaynak et al., EMBO J., 9, pp. 2085-2093 (1990); OP-3 (WO94/10203 (PCT US93/10520)), BMP-2, BMP-4, (WO88/00205; Wozney et al. Science, 242, pp. 1528-1534 (1988)), BMP-5 and BMP-6, (Celeste et al., PNAS, 87, 9843-9847 (1990)), Vgr-1 (Lyons et al., PNAS, 86, pp. 4554-4558 (1989)); DPP (Padgett et al. Nature, 325, pp. 81-84 (1987)); Vg-1 (Weeks, Cell, 51, pp. 861-867 (1987)); BMP-9 (WO95/33830 (PCT/US95/07084); BMP-10 (WO94/26893 (PCT/US94/05290); BMP-11 (WO94/26892 (PCT/US94/05288); BMP-12 (WO95/16035 (PCT/US94/14030); BMP-13 (WO95/16035 (PCT/US94/14030); GDF-1 (WO92/00382 (PCT/US91/04096) and Lee et al. PNAS, 88, pp. 4250-4254 (1991); GDF-8 (WO94/21681 (PCT/US94/03019); GDF-9 (WO94/15966 (PCT/US94/00685); GDF-10 (WO95/10539 (PCT/US94/11440); GDF-11 (WO96/01845 (PCT/US95/08543); BMP-15 (WO96/36710 (PCT/US96/06540); MP-121 (WO96/01316 (PCT/EP95/02552); GDF-5 (CDMP-1, MP52) (WO94/15949 (PCT/US94/00657) and WO96/14335 (PCT/US94/12814) and WO93/16099 (PCT/EP93/00350)); GDF-6 (CDMP-2, BMP13) (WO95/01801 (PCT/US94/07762) and WO96/14335 and WO95/10635 (PCT/US94/14030)); GDF-7 (CDMP-3, BMP12) (WO95/10802 (PCT/US94/07799) and WO95/10635 (PCT/US94/14030)) The above publications are incorporated herein by reference.

As used herein, "TGF-β superfamily member" or "TGF-β superfamily protein," means a protein known to those of ordinary skill in the art as a member of the Transforming Growth Factor- β (TGF-β) superfamily. Structurally, such proteins are homo or heterodimers expressed as large precursor polypeptide chains containing a hydrophobic signal sequence, an N-terminal pro region of several hundred amino acids, and a mature domain comprising a variable N-terminal region and a highly conserved C-terminal region containing approximately 100 amino acids with a characteristic cysteine motif having a conserved six or seven cysteine skeleton. These structurally-related proteins have been identified as being involved in a variety of developmental events.

The term "morphogenic protein" refers to a protein belonging to the TGF-ß superfamily of proteins which has true morphogenic activity. For instance, such a protein is capable of inducing progenitor cells to proliferate and/or to initiate a cascade of events in a differentiation pathway that leads to the formation of cartilage, bone, tendon, ligament, neural or other types of differentiated tissue, depending on local environmental cues. Thus, morphogenic proteins useful in this invention can behave differently in different surroundings. In certain embodiments, a morphogenic protein of this invention can be a homodimer species or a heterodimer species.

The term "osteogenic protein (OP)" refers to a morphogenic protein that is also capable of inducing a progenitor cell to form cartilage and/or bone. The bone can be intramembranous bone or endochondral bone. Most osteogenic proteins are members of the BMP subfamily and are thus also BMPs. However, the converse can not be true. According to this invention, a BMP identified by DNA sequence homology or amino acid sequence identity must also have demonstrable osteogenic or chondrogenic activity in a functional bioassay to be an osteogenic protein. Appropriate bioassays are well known in the art; a particularly useful bioassay is the heterotopic bone formation assay (see, U.S. Pat. No. 5,011,691; U.S. Pat. No. 5,266,683, for example).

BMPs are naturally expressed as pro-proteins comprising a long pro-domain, one or more cleavage sites, and a mature domain. This pro-protein is then processed by the cellular machinery to yield a dimeric mature BMP molecule. The pro-domain is believed to aid in the correct folding and processing of BMPs. Furthermore, in some but not all BMPs, the pro-domain can noncovalently bind the mature domain and can act as a chaperone, as well as an inhibitor (e.g., Thies et. al. (2001) Growth Factors, 18:251-259).

Structurally, BMPs are dimeric cysteine knot proteins. Each BMP monomer comprises multiple intramolecular disulfide bonds. An additional intermolecular disulfide bond mediates dimerization in most BMPs. BMPs can form homodimers. Some BMPs can form heterodimers.

BMP signal transduction is initiated when a BMP dimer binds two type I and two type II serine/threonine kinase receptors. Type I receptors include, but are not limited to, ALK-1, ALK-2 (also called ActRla or ActRI), ALK-3 (also called BMPRIa), and ALK-6 (also called BMPRIb). Type II receptors include, but are not limited to, ActRIIa (also called ActRII), ActRIIb, and BMPRII. Human genome contains 12 members of the receptor serine/threonine kinase family, including 7 type I and 5 type II receptors, all of which are involved in TGF-β signaling (Manning et al., 2002, the disclosures of which are hereby incorporated by reference). Following BMP binding, the type II receptors phosphorylate the type I receptors, the type I receptors phosphorylate members of the Smad family of transcription factors, and the Smads translocate to the nucleus and activate the expression of a number of genes.

BMPs also interact with inhibitors, soluble receptors, and decoy receptors, including, but not limited to, BAMBI (BMP and activin membrane bound inhibitor), BMPER (BMP-binding endothelial cell precursor-derived regulator), Cerberus, cordin, cordin-like, Dan, Dante, follistatin, follistatin-related protein (FSRP), ectodin, gremlin, noggin, protein related to Dan and cerberus (PRDC), sclerostin, sclerostin-like, and uterine sensitization-associated gene-1 (USAG-1). Furthermore, BMPs can interact with co-receptors, for example BMP-2 and BMP-4 bind the co-receptor DRAGON (Samad et. al. (2005) J. Biol. Chem., 280:14122-14129), and extracellular matrix components such as heparin sulfate and heparin (Irie et al. (2003) Biochem. Biophys. Res. Commun. 308: 858-865).

As contemplated herein, the term "BMP" refers to a protein belonging to the BMP subfamily of the TGF-β superfamily of proteins defined on the basis of DNA homology and amino acid sequence identity. According to this invention, a protein belongs to the BMP subfamily when it has at least 50% amino acid sequence identity with a known BMP subfamily member within the conserved C-terminal cysteine-rich domain that characterizes the BMP subfamily. Members of the BMP subfamily can have less than 50% DNA or amino acid sequence identity overall. As used herein, the term "BMP" further refers to proteins which are amino acid sequence variants, domain-swapped variants, and truncations and active fragments of naturally occurring bone morphogenetic proteins, as well as heterodimeric proteins formed from two different monomeric BMP peptides, such as BMP-2/7; BMP-4/7: BMP-2/6; BMP-2/5; BMP-4/7; BMP-4/5; and BMP-4/6 heterodimers. Suitable BMP variants and heterodimers include those set forth in US 2006/0235204; WO 07/087053; WO 05/097825; WO 00/020607; WO 00/020591; WO 00/020449; WO 05/113585; WO 95/016034 and WO93/009229.

According to one embodiment, a BMP used in a formulation according to the invention can maintain at least 80%, at least 81%, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, at least 98%, or at least 99% identity with the corresponding wild-type BMP protein sequence.

According to one embodiment, a BMP used in a formulation according to the invention can maintain at least 80%, at least 81%, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, at least 98%, or at least 99% identity with the conserved cysteine domain of the C-terminal region of the corresponding wild-type BMP protein sequence.

By "corresponding wild-type protein" it is meant the wild-type version of the modified BMP. For example, if the modified BMP is a modified BMP-7, the corresponding wild-type BMP is wild-type BMP-7.

To determine the percent identity of two amino acid sequences or of two nucleic acids, the sequences are aligned for optimal comparison purposes (e.g., gaps can be introduced in the sequence of a first amino acid or nucleic acid sequence for optimal alignment with a second amino acid or nucleic acid sequence). The percent identity between the two sequences is a function of the number of identical positions shared by the sequences (i.e., % homology=# of identical positions/total # of positionsX100). The determination of percent homology between two sequences can be accomplished using a mathematical algorithm. A preferred, non-limiting example of a mathematical algorithm utilized for the comparison of two sequences is the algorithm of Karlin and Altschul (1990) Proc. Natl. Acad. Sci. USA 87:2264-68, modified as in Karlin and Altschul (1993) Proc. Natl. Acad. Sci. USA 90:5873-77. Such an algorithm is incorporated into the NBLAST and XBLAST programs of Altschul, et al. (1990) J. Mo/. Biol. 215:403-10. BLAST nucleotide searches can be performed with the NBLAST program, score=100, wordlength=12. BLAST protein searches can be performed with the XBLAST program, score=50, wordlength=3. To obtain gapped alignments for comparison purposes, Gapped BLAST can be utilized as described in Altschul et al., (1997) Nucleic Acids Research 25(17):3389-3402. When utilizing BLAST and Gapped BLAST programs, the default parameters of the respective programs (e.g., XBLAST and NBLAST) can be used.

### Therapeutic Uses

The BMP formulations of the invention, in solid form, can be implanted in a mammalian patient, for example, a human to treat a wide variety of conditions. BMP formulations of the invention can be implanted in solid, gel or paste form, or injected into the patient in a gel, paste or liquid form.

The BMP formulations of the invention are useful for treating a wide variety of conditions. For example, the formulations containing BMPs can be used to treat skeletal disorders, including cartilage degeneration whether caused by trauma or inflammatory disease. For example, diseases treatable by formulations of the invention include rheumatoid arthritis (RA) and osteoarthritis (OA) and autoimmune diseases such as systemic lupus erythematosis (SLE) and scleroderma.

The BMP formulations of the invention can be used effectively to treat skeletal diseases or injuries. For example, the formulations can be used to treat a bone fracture, such as an open fracture or a closed fracture. For the treatment of a closed fracture, the formulation is preferably injected at the fracture site. For open fractures, critical size defects or persistent nonunions, the formulations can be administered by surgical implantation at the fracture site. In both cases, the formulation can be administered alone, or in combination with a suitable carrier, matrix or scaffold, such as a bone cement, a calcium phosphate material, a gel material or a collagen matrix. Suitable carriers, matrices and scaffolds include those disclosed in US Patent Nos. 6,919,308; 6,949,251; and 7,041,641.

In a preferred embodiment, the BMP formulations of the invention can be used to treat a disease or injury resulting in cartilage degradation or a cartilage defect. For example, the formulations can be applied to a cartilage defect site, such as a degenerative intervertebral disc, or other fibrocartilaginous tissue, including a tendon, a ligament or a meniscus. Such methods are set out in U.S. Patent No. 6,958,149. The formulations of the invention can also be used to treat a defect or degeneration of articular cartilage, as set forth in published PCT application WO 05/115438, such as the cartilage lining of a joint, such as a synovial joint, including a knee, an elbow, a hip, or a shoulder. In this embodiment, the formulation is preferably injected into the synovial space of the joint. In another embodiment, the formulations of the invention are used to treat an articular cartilage defect site, such as a chondral defect or an osteochondral defect, in a joint. Such articular cartilage defects can be the result of a disease process, such as osteoarthritis or rheumatoid arthritis, or due to injury of the joint. In this embodiment, the formulation can be injected into the joint space or it can be surgically implanted. For example, the formulation can be placed within the defect either alone or in combination with one or more additional active agents, a supporting matrix or scaffold, or marrow stromal cells. The formulation can, optionally, be covered with a suitable covering, for example a muscle flap or a bioresorbable membrane, such as a collagen membrane.

As will be appreciated by those skilled in the art, the concentration of the compounds described in a therapeutic composition will vary depending upon a number of factors, including without limitation the dosage of the drug to be administered and the route of administration. The preferred dosage of drug to be administered also is likely to depend on variables including, but not limited to, the type and extent of a disease, tissue loss or defect, the overall health status of the particular patient, the relative biological efficacy of the compound selected, the formulation of the compound, the presence and types of excipients in the formulation, and the route of administration. The present invention can be provided to an individual where typical doses range from about 10 ng/kg to about 1 g/kg of body weight per day; with a preferred dose range being from about 0.1 mg/kg to 100 mg/kg of body weight, and with a more particularly preferred dosage range of 10-1000 µg/dose. In a particularly preferred embodiment, a dose of 10-1000 µg of a BMP-7 is administered to an individual afflicted with osteoarthritis.

Additionally, as described below, the protein formulations, preferably the BMP formulations of the present invention can be used to treat diseases or injuries of non-skeletal tissues. As further contemplated by the present invention, BMPs are capable of inducing the developmental cascade of bone morphogenesis and tissue morphogenesis for a variety of tissues in mammals different from bone or bone cartilage. This morphogenic activity includes the ability to induce proliferation and differentiation of progenitor cells, and the ability to support and maintain the differentiated phenotype through the progression of events that results in the formation of bone, cartilage, non-mineralized skeletal or connective tissues, and other adult tissues.

For example, BMPs can be used for treatment to prevent loss of and/or increase bone mass in metabolic bone diseases. General methods for treatment to prevent loss of and/or increase bone mass in metabolic bone diseases using osteogenic proteins are disclosed in U.S. Patent No. 5,674,844, the disclosures of which are hereby incorporated by reference. BMPs of the present invention can be used for periodontal tissue regeneration. General methods for periodontal tissue regeneration using osteogenic proteins are disclosed in U.S. Patent No. 5,733,878, the disclosures of which are hereby incorporated by reference. BMPs can be used for liver regeneration. General methods for liver regeneration using osteogenic proteins are disclosed in U.S. Patent No. 5,849,686, the disclosures of which are hereby incorporated by reference. BMPs can be used for treatment of chronic renal failure. General methods for treatment of chronic renal failure using osteogenic proteins are disclosed in U.S. Patent No. 6,861,404, the disclosures of which are hereby incorporated by reference. BMPs can be used for enhancing functional recovery following central nervous system ischemia or trauma. General methods for enhancing functional recovery following central nervous system ischemia or trauma using osteogenic proteins are disclosed in U.S. Patent No. 6,407,060, the disclosures of which are hereby incorporated by reference. BMPs can be used for inducing dendritic growth. General methods for inducing dendritic growth using osteogenic proteins are disclosed in U.S. Patent No. 6,949,505, the disclosures of which are hereby incorporated by reference. BMPs can be used for inducing neural cell adhesion. General methods for inducing neural cell adhesion using osteogenic proteins are disclosed in U.S. Patent No. 6,800,603, the disclosures of which are hereby incorporated by reference. BMPs can be used for treatment and prevention of Parkinson's disease. General methods for treatment and prevention of Parkinson's disease using osteogenic proteins are disclosed in U.S. Patent No. 6,506,729, the disclosures of which are hereby incorporated by reference.

As another example, BMPs can also be used to induce dentinogenesis. To date, the unpredictable response of dental pulp tissue to injury is a basic clinical problem in dentistry. As yet another example, BMPs can induce regenerative effects on central nervous system (CNS) repair can be assessed using a rat brain stab model.

### Example 1. Glycylglycine buffer and tartaric acid buffer stabilizes the pH of BMP formulations at increasing concentrations of BMP.

To evaluate the potential for buffers to mitigate the pH increase observed in highly concentrated BMP formulations, six buffers were tested using the exemplary BMP, BMP-7: tartaric acid (pKa 2.98, 4.34), glycylglycine (pKa 3.14), malic acid (pKa 3.40), lactic acid (pKa 3.86), aspartic acid (pKa 3.90), and succinic acid (pKa 4.21).

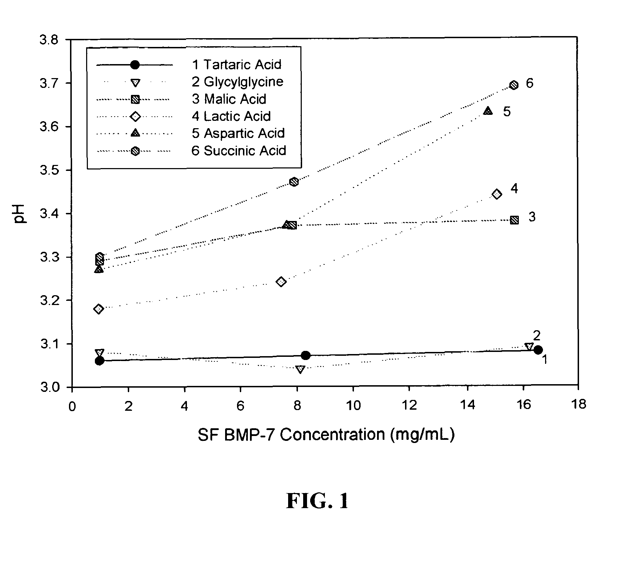

Each buffer was prepared at a concentration of 10 mM and the pH was adjusted with 1 N HCl or 1 N NaOH as needed to yield a final pH of 3.0. Each buffer also contained 9% trehalose. BMP-7 in 50 mM acetic acid at 1.6 mg/mL was concentrated to 10 mg/mL and then dialyzed into each of the six buffers. After dialysis, the concentration of the protein in each buffer was 15 to 16 mg/mL. The concentration of the protein and the pH at room temperature were measured for each buffer. Subsequently, each of the protein solutions were diluted to 8 mg/mL and 1 mg/mL with their respective buffers. The pH and protein concentration at room temperature were measured at each of the diluted concentrations. The results for pH as a function of BMP-7 concentration are shown in FIG. 1.

As shown in FIG. 1, an undesirable increase in pH is observed for the malic acid, lactic acid, aspartic acid, and succinic acid buffers as concentrations of BMP-7 increase. In contrast, no significant increase was observed in the tartaric acid or glycylglycine buffers as the concentration of BMP-7 increased. Accordingly, either tartaric acid or glycylglycine can be used as buffers in order to mitigate pH increases resulting from addition of BMP-7 to the buffer.

### Example 2. Glycylglycine buffers and tartaric acid buffers lend stability to BMP liquid formulations over time by reducing aggregation.

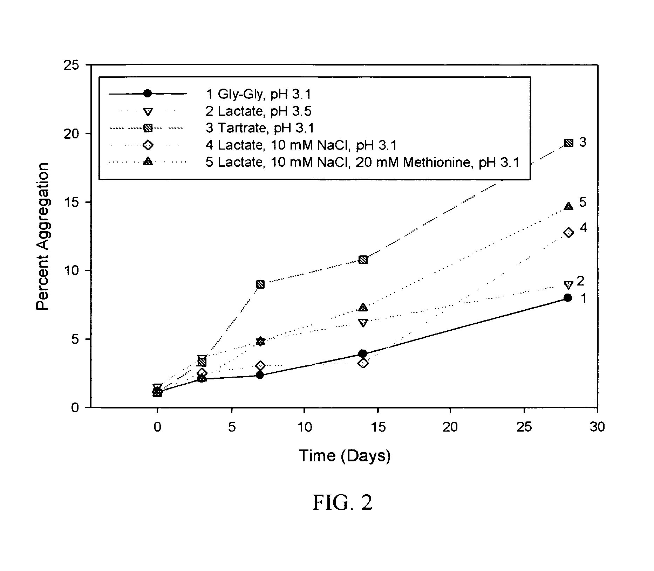

A liquid stability study was conducted to evaluate protein aggregation in BMP formulations using the exemplary BMP, BMP-7, with buffers of 10 mM glycylglycine (pH 3.1), 10 mM tartrate (pH 3.1), 10 mM lactate (pH 3.5), 10 mM lactate with 10 mM NaCl (pH 3.1), and 10 mM lactate with 10 mM NaCl and 20 mM methionine (pH 3.1). Formulations that minimize changes in the protein, including aggregation, provide improved stability and consistency of lyophilized preparations. The BMP-7 concentration was 16 mg/mL. The formulations were held under accelerated stability conditions at 40°C for 4 weeks. The percent aggregation of BMP-7 in the liquid formulations was measured by size exclusion chromatography and the results are shown in FIG. 2.

As shown in FIG. 2, the formulations with glycylglycine buffer at pH 3.1 exhibited the lowest rate of aggregation increase over the 4 week period, indicating that the glycylglycine provides the greatest stability to BMP-7 formulations than the other buffers tested.

### Example 3. Glycylglycine buffers and tartaric acid buffers lend stability to BMP lyophilized formulations over time by reducing aggregation.

An accelerated stability study of both lyophilized liquid and lyophilized BMP and buffer formulations was conducted to evaluate BMP aggregation in formulations with buffers of 10 mM glycylglycine HCl (pH 3.1), 10 mM tartrate (pH 3.1), 10 mM lactate (pH 3.1), 10 mM lactate with 10 mM NaCl (pH 3.1), and 10 mM lactate with 10 mM NaCl and 20 mM methionine (pH 3.1) using the exemplary BMP, BMP-7. The BMP-7 concentration was either I mg/mL or 16 mg/mL. The formulations for the liquid stability study were dispensed into vials and held at 40°C for 2 months or lyophilized and held at 40°C for 6 months.

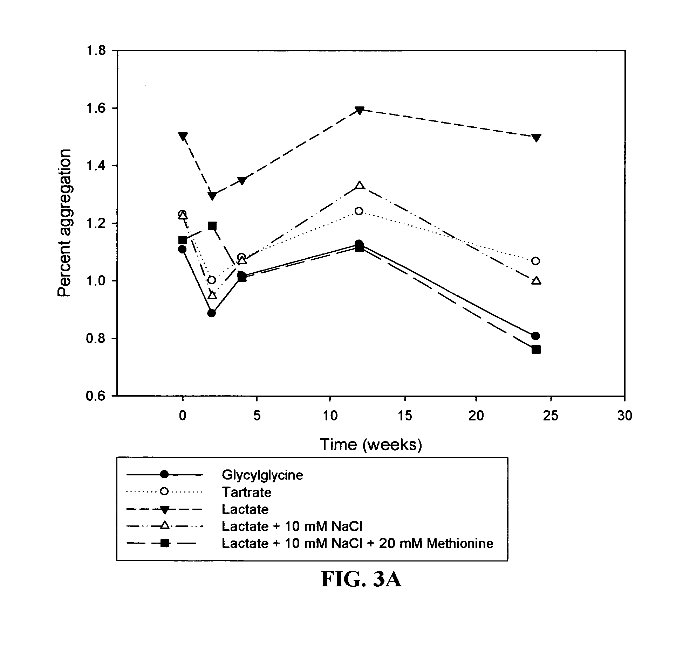

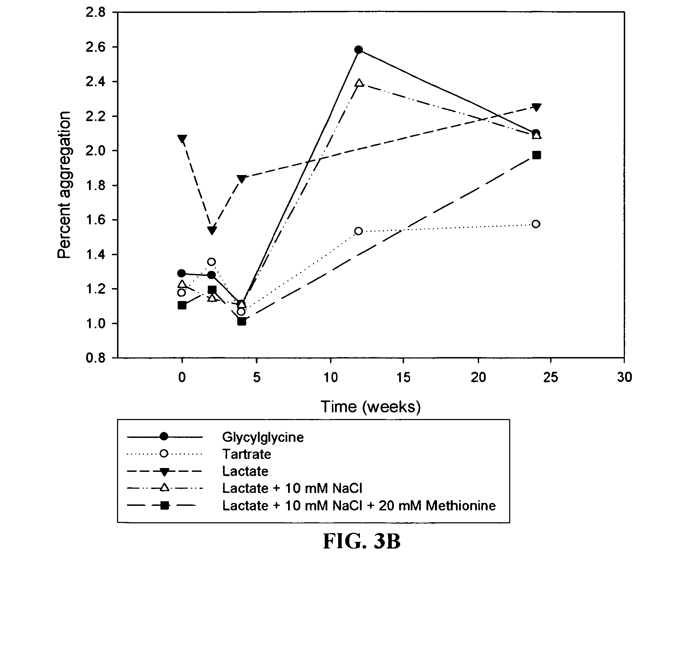

As shown in FIG. 3A, BMP-7 formulations containing lactate with methionine and glycylglycine had the lowest aggregation rates for those formulation buffers at 1 mg/mL at 6 months. As shown in FIG. 3B, at 16 mg/mL, there was an increase observed in protein aggregation for all buffers tested. The lowest aggregation was seen in the formulation containing tartrate. The example demonstrated improved stability associated with BMP-7 formulations containing 10 mM glycylglycine, 9% trehalose, pH 3.0 and BMP-7 formulations containing 10 mM tartrate, 9% trehalose, pH 3.0 compared to formulations containing 10 mM lactate.

### Example 4. BMPs from Glycylglycine and tartaric acid buffer formulations have biological activity.

The ability of BMP from a solution of BMP and glycylglycine buffer or tartaric acid buffer that has been held at 40°C for 6 months to induce alkaline phosphatase (ALP) activity in the rat osteosarcoma cell line ROS 17/2.8 is assayed. The exemplary BMP, BMP-7, is tested in a nine point dose response in triplicate. In particular, ROS 17/2.8 cells are plated in 96-well tissue culture plates. BMP from the glycylglycine solution or the tartaric acid solution is added to the cells in the following dosages: 6000, 2000, 666, 222, 74, 24, 8, 2, and 0.9 ng/ml and incubated for a period of 48 hours. Cells are subsequently lysed and potency of BMP-7 to induce ALP activity is assessed based on the EC50 derived from non-linear regression of the mean optical density (OD) of the samples. The exemplary BMP, BMP-7, from the glycylglycine solution and the tartaric acid solution both demonstrate robust biological activity.

### Example 5. BMP Formulations containing Glycylglycine buffer

A formulation according to the invention is prepared by performing a buffer exchange by dialysis, tangential flow filtration, or ultrafiltration/diafiltration (UF/DF) process with BMP, for example BMP-7. 100 mg of BMP-7 in 10 mL of dialysis buffer is dialyzed against 300 mL of 10 mM glycylglycine buffer, 9% trehalose, and 20 mM methionine at pH 3.0±0.2 with 3 changes of dialysis buffer. The glycylglycine buffer is prepared by mixing 500 mL of 10 mM glycylglycine-HCl solution at pH 2.6 with 210 mL of 10 mM glycylglycine at pH 5.7. The resulting concentration of BMP-7 is adjusted to 1 mg/mL (about 100 mL) using dialysis buffer and 0.01% polysorbate 20 or 0.01 % polysorbate 80 is added with a final pH of 3.0±0.2. Similarly, about 100 mg to 10 g of BMP-7 in about 10 mL to 1000 mL dialysis buffer is exchanged against about 10x volume of 10 mM glycylglycine buffer via UF/DF. Additional formulation constituents, such as trehalose, methionine, and/or polysorbate 20 are spiked in to desired amounts. The formulation is diluted to the desired concentration using the formulation buffer and the final formulation pH is 3.0±0.2.

### Example 6. BMP Formulations containing tartaric acid buffer

A formulation according to the invention is prepared by performing buffer exchange by dialysis or tangential flow filtration with BMP, for example BMP-7. 100 mg of BMP-7 in 10 mL dialysis buffer is dialyzed against 300 mL of 10mM tartaric acid buffer, 9% trehalose, and 20 mM methionine at a pH of 3.0±0.2, with 3 changes of dialysis buffer. The tartaric acid buffer is prepared by mixing 840mL of 10 mM tartaric acid solution with 310 mL of 10 mM potassium sodium tartrate at pH7.2. The resulting concentration of BMP is adjusted to 1 mg/mL (about 100 mL) using dialysis buffer and 0.0 1 % polysorbate 20 or 0.01 % polysorbate 80 is spiked in with a final pH of 3.0±0.2. Similarly, about 100 mg to about 10 g of BMP-7 in about 10 mL to 1000 mL is exchanged against about 10x volume of 10 mM tartrate buffer via UF/DF. Additional formulation constituents such as trehalose, methionine, and/or polysorbate 20 are spiked in to desired amounts. The formulation is diluted to the desired concentration using the formulation buffer and the final formulation is pH 3.0±0.2.

### Example 7. BMP Formulations containing tartaric acid buffer and trehalose

As demonstrated herein, glycylglycine and tartrate control the pH of BMP-7 formulations more effectively than lactate and may be advantageous in formulating drug product. In order to assess stability of lyophilized BMP-7 in glycylglycine and tartrate, long term lyophilized stability studies were initiated.

Three lots of BMP-7 were formulated at 1 mg/mL in either10 mM lactate, 9% trehalose, pH 3.0; 10 mM glycylglycine, 9% trehalose, pH 3.0; or 10 mM tartrate, 9% trehalose, pH 3.0. The pH of all formulations was controlled within 3.0±0.2. The formulated drug product was lyophilized and long term stability studies were initiated at 5°C or 30°C. Stability of the three lots was evaluated through 2, 6, and 9 months.

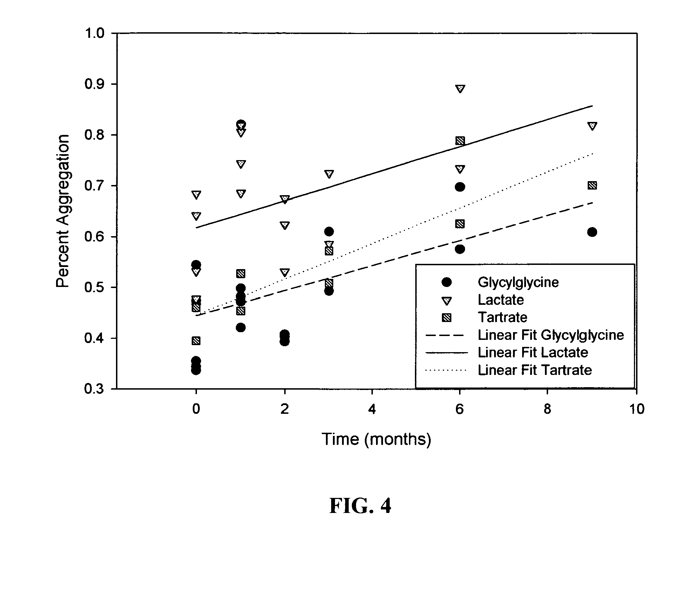

BMP-7 aggregation in the formulation containing lactate was initially higher than in glycylglycine or tartrate. There were no relevant changes in aggregation at 5°C for any of the formulations. Aggregation increased for all formulations at 30°C with the lowest aggregation observed in the formulation containing glycylglycine (see FIG. 4). The results suggest that BMP-7 formulations containing 10 mM glycylglycine or 10 mM tartrate can improve the stability of a lyophilized drug product compared to buffering with 10 mM lactate.

## Claims

1. A solution comprising a bone morphogenetic protein and an aqueous glycylglycine buffer.

2. The solution of claim 1 wherein the protein concentration is:
(i) from about 1 mg/mL to about 20 mg/mL;
(ii) from about 0.1 mg/mL to about 20 mg/mL;
(iii) from about 0.1 mg/mL to about 1 mg/mL; or
(iv) from about 0.1 mg/mL to about 40 mg/mL.

3. A solid composition comprising:
(i) a bone morphogenetic protein; and
(ii) glycylglycine, a glycylglycine salt, or a combination thereof.

4. The solid composition of claim 3, wherein:
(i) the glycylglycine salt is glycylglycine HCl; and/or
(ii) the ratio of bone morphogenetic protein to glycylglycine, glycylglycine salt, or combination thereof is from about 8x 10⁻⁴: 1 to about 310: 1 (w/w).

5. A solution comprising a bone morphogenetic protein and an aqueous tartaric acid buffer.

6. The solution of claim 5 wherein the protein concentration is:
(i) from about 0.01 mg/mL to about 40 mg/mL;
(ii) from about 1 mg/mL to about 20 mg/mL;
(iii) from about 0.1 mg/mL to about 1 mg/mL; or
(iv) from about 0.1 mg/mL to about 20 mg/mL.

7. A solid composition comprising:
(i) a bone morphogenetic protein; and
(ii) tartaric acid, a tartrate salt or a combination thereof,
optionally wherein the ratio of bone morphogenetic protein to tartaric acid, tartrate salt, or combination thereof is from about 7x 10⁻⁴: 1 to about 270: 1 (w/w).

8. The solution or composition of any one of the preceding claims, wherein the bone morphogenetic protein is selected from BMP-2, BMP-4, BMP-5, BMP-6, BMP-7, GDF-5, GDF-6 and GDF-7.

9. The solution of any one of claims 1, 2, 5, 6 or 8, wherein the pH of the solution is from about:
(i) 2.5 to about 4.0;
(ii) 2.8 to about 3.5; or
(iii) 2.8 to about 3.2.

10. The solution of any one of claims 1, 2, 5, 6, 8 or 9, wherein the buffer has a glycylglycine or tartaric acid concentration:
(i) from about 1 mM to about 100 mM;
(ii) from about 2 mM to about 20 mM;
(iii) from about 5 mM to about 15 mM; or
(iv) of about 10 mM.

11. The solution of any one of claims 1, 2, 5, 6, 8, 9 or 10, wherein the pH does not vary more than about:
(i) 0.2 pH units upon storage at 40°C for six months; or
(ii) 0.2 pH units upon storage at 40°C for 36 months.

12. The solution or composition of any one of the preceding claims, further comprising a lyoprotectant, optionally wherein the lyoprotectant is a sugar.

13. The solution or composition of claim 12, wherein the lyoprotectant is selected from mannitol, lactose, sucrose and trehalose and combinations thereof.

14. The solution or composition of claim 13, wherein the lyoprotectant is trehalose and:
(i) the trehalose is present in an amount from about 1% to about 15% (w/v);
(ii) the trehalose is present in an amount from about 3% to about 9%, e.g. about 9%; or
(iii) the ratio of bone morphogenetic protein to trehalose is from about 7x 10⁻⁵: 1 to about 1.3: 1 (w/w).

15. The solution or composition of any one of the preceding claims further comprising an antioxidant, optionally selected from methionine, ascorbic acid, benzyl alcohol, glutathione, m-cresol and thioglycerol.

16. A solid composition obtainable by a method comprising the step of lyophilizing the solution of any one of claims 1, 2, 5, 6, 8, 9, 10, 11, 12, 13, 14 or 15.

17. A solution according to claim 1 or claim 5, further comprising a stabilizer selected from proline, glycine, valine, isoleucine, and leucine, optionally wherein the stabilizer comprises proline.

18. A solid composition comprising:
(i) a bone morphogenetic protein;
(ii) a glycylglycine salt or tartrate salt; and
(iii) a compound selected from proline, glycine, valine, isoleucine, leucine, a salt of proline, a salt of glycine, a salt of valine, a salt of isoleucine, or a salt of leucine.

## Patentansprüche

1. Lösung, die ein knochenmorphogenetisches Protein (Bone Morphogenetic Protein - BMP) und einen wässrigen Glycylglycinpuffer enthält.

2. Lösung gemäß Anspruch 1, wobei die Proteinkonzentration folgendermaßen ist:
(i) von etwa 1 mg/ml bis etwa 20 mg/ml;
(ii) von etwa 0,1 mg/ml bis etwa 20 mg/ml;
(iii) von etwa 0,1 mg/ml bis etwa 1 mg/ml; oder
(iv) von etwa 0,1 mg/ml bis etwa 40 mg/ml.

3. Feste Zusammensetzung, die Folgendes enthält:
(i) ein knochenmorphogenetisches Protein (Bone Morphogenetic Protein - BMP); und
(ii) Glycylglycin, ein Glycylglycinsalz oder eine Kombination von diesen.

4. Feste Zusammensetzung gemäß Anspruch 3, wobei:
(i) es sich bei dem Glycylglycinsalz um Glycylglycin-HCl handelt; und/oder
(ii) das Verhältnis des knochenmorphologischen Proteins zum Glycylglycin, dem Glycylglycinsalz oder der Kombination von diesen zwischen etwa 8 x 10⁻⁴ : 1 und etwa 310 : 1 (Massenanteil) beträgt.

5. Lösung, die ein knochenmorphogenetisches Protein (Bone Morphogenetic Protein - BMP) und einen wässrigen Weinsäurepuffer enthält.

6. Lösung gemäß Anspruch 5, wobei die Proteinkonzentration folgendermaßen ist:
(i) von etwa 0,01 mg/ml bis etwa 40 mg/ml;
(ii) von etwa 1 mg/ml bis etwa 20 mg/ml;
(iii) von etwa 0,1 mg/ml bis etwa 1 mg/ml; oder
(iv) von etwa 0,1 mg/ml bis etwa 20 mg/ml.

7. Feste Zusammensetzung, die Folgendes enthält:
(i) ein knochenmorphogenetisches Protein (Bone Morphogenetic Protein - BMP); und
(ii) Weinsäure, ein Tartratsalz oder eine Kombination von diesen,
wobei optional das Verhältnis des knochenmorphologischen Proteins zur Weinsäure, dem Tartratsalz oder der Kombination von diesen zwischen etwa 7 x 10⁻⁴ : 1 und etwa 270 : 1 (Massenanteil) beträgt.

8. Lösung oder Zusammensetzung gemäß einem der vorherigen Ansprüche, wobei das knochenmorphogenetische Protein aus BMP-2, BMP-4, BMP-5, BMP-6, BMP-7, GDF-5, GDF-6 und GDF-7 ausgewählt ist.

9. Lösung gemäß einem der Ansprüche 1, 2, 5, 6 oder 8, wobei der pH-Wert der Lösung in folgenden Bereichen liegt:
(i) von etwa 2,5 bis etwa 4,0;
(ii) von etwa 2,8 bis etwa 3,5; oder
(iii) von etwa 2,8 bis etwa 3,2.

10. Lösung gemäß einem der Ansprüche 1, 2, 5, 6, 8 oder 9, wobei der Puffer die folgende Glycylglycin- bzw. Weinsäurekonzentration aufweist:
(i) von etwa 1 mM bis etwa 100 mM;
(ii) von etwa 2 mM bis etwa 20 mM;
(iii) von etwa 5 mM bis etwa 15 mM; oder
(iv) etwa 10 mM.

11. Lösung gemäß einem der Ansprüche 1, 2, 5, 6, 8, 9 or 10, wobei der pH-Wert um nicht mehr als etwa die folgenden Werte schwankt:
(i) 0,2 pH-Einheiten bei Lagerung bei 40 °C über sechs Monate hinweg; oder
(ii) 0,2 pH-Einheiten bei Lagerung bei 40 °C über 36 Monate hinweg.

12. Lösung oder Zusammensetzung gemäß einem der vorherigen Ansprüche, die des Weiteren einen Lyoprotektor enthält, wobei es sich bei dem Lyoprotektor optional um einen Zucker handelt.

13. Lösung oder Zusammensetzung gemäß Anspruch 12, wobei der Lyoprotektor aus Mannitol, Lactose, Saccharose und Trehalose und Kombinationen von diesen ausgewählt ist.

14. Lösung oder Zusammensetzung gemäß Anspruch 13, wobei es sich bei dem Lyoprotektor um Trehalose handelt und:
(i) die Trehalose mit einem Anteil von etwa 1 % bis etwa 15 % (Volumenanteil) vorhanden ist;
(ii) die Trehalose mit einem Anteil von etwa 3 % bis etwa 9 % vorhanden ist, z. B. etwa 9 %; oder
(iii) das Verhältnis des knochenmorphologischen Proteins zur Trehalose zwischen etwa 7 x 10⁻⁵ : 1 und etwa 1,3 : 1 (Massenanteil) beträgt.

15. Lösung oder Zusammensetzung gemäß einem der vorherigen Ansprüche, die des Weiteren ein Antioxidationsmittel enthält, das optional ausgewählt ist aus Methionin, Ascorbinsäure, Benzylalkohol, Glutathion, m-Kresol und Thioglycerol.

16. Feste Zusammensetzung, die durch ein Verfahren gewonnen werden kann, das den Schritt der Lyophilisation der Lösung gemäß einem der Ansprüche 1, 2, 5, 6, 8, 9, 10, 11, 12, 13, 14 oder 15 umfasst.

17. Lösung gemäß Anspruch 1 oder Anspruch 5, die des Weiteren einen Stabilisator enthält, der aus Prolin, Glycin, Valin, Isoleucin und Leucin ausgewählt ist, wobei der Stabilisator optional Prolin enthält.

18. Feste Zusammensetzung, die Folgendes enthält:
(i) ein knochenmorphogenetisches Protein (Bone Morphogenetic Protein - BMP);
(ii) ein Glycylglycinsalz oder ein Tratratsalz; und
(iii) eine Verbindung, die aus Prolin, Glycin, Valin, Isoleucin, Leucin, einem Prolinsalz, einem Glycinsalz, einem Valinsalz, einem Isoleucinsalz oder einem Leucinsalz ausgewählt ist.

## Revendications

1. Une solution comportant une protéine morphogénétique osseuse et un tampon glycylglycine aqueux.

2. La solution de la revendication 1 dans laquelle la concentration protéinique est :
(i) d'environ 1 mg/mL à environ 20 mg/mL ;
(ii) d'environ 0,1 mg/mL à environ 20 mg/mL ;
(iii) d'environ 0,1 mg/mL à environ 1 mg/mL ; ou
(iv) d'environ 0,1 mg/mL à environ 40 mg/mL.

3. Une composition solide comportant :
(i) une protéine morphogénétique osseuse ; et
(ii) glycylglycine, un sel de glycylglycine, ou une combinaison de ces derniers.

4. La composition solide de la revendication 3, dans laquelle :
(i) le sel de glycylglycine est glycylglycine HCl ; et/ou
(ii) le ratio de protéine morphogénétique osseuse à glycylglycine, sel de glycylglycine, ou combinaison de ces derniers est d'environ 8x 10⁻⁴:1 à environ 310: 1 (M/M).

5. Une solution comportant une protéine morphogénétique osseuse et un tampon acide tartarique aqueux.

6. La solution de la revendication 5 dans laquelle la concentration protéinique est :
(i) d'environ 0,01 mg/mL à environ 40 mg/mL ;
(ii) d'environ 1 mg/mL à environ 20 mg/mL ;
(iii) d'environ 0,1 mg/mL à environ 1 mg/mL ; ou
(iv) d'environ 0,1 mg/mL à environ 20 mg/mL.

7. Une composition solide comportant :
(i) une protéine morphogénétique osseuse ; et
(ii) acide tartarique, un sel de tartrate ou une combinaison de ces derniers,
optionnellement dans laquelle le ratio de protéine morphogénétique osseuse à acide tartarique, sel de tartrate, ou combinaison de ces derniers est d'environ 7x 10⁻⁴: 1 à environ 270: 1 (M/M).

8. La solution ou composition de n'importe laquelle des revendications précédentes, dans laquelle la protéine morphogénétique osseuse est sélectionnée parmi BMP-2, BMP-4, BMP-5, BMP-6, BMP-7, GDF-5, GDF-6 et GDF-7.

9. La solution de n'importe laquelle des revendications 1, 2, 5, 6 ou 8, dans laquelle le pH de la solution est d'environ :
(i) 2,5 à environ 4,0 ;
(ii) 2,8 à environ 3,5 ; ou
(iii) 2,8 à environ 3,2.

10. La solution de n'importe laquelle des revendications 1, 2, 5, 6, 8 ou 9, dans laquelle le tampon a une concentration en glycylglycine ou acide tartarique :
(i) d'environ 1 mM à environ 100 mM ;
(ii) d'environ 2 mM à environ 20 mM ;
(iii) d'environ 5 mM à environ 15 mM ; ou
(iv) d'environ 10 mM.

11. La solution de n'importe laquelle des revendications 1, 2, 5, 6, 8, 9 ou 10, dans laquelle le pH ne varie pas de plus d'environ :
(i) 0,2 unité de pH après stockage à 40 °C pendant six mois ; ou
(ii) 0,2 unité de pH après stockage à 40 °C pendant 36 mois.

12. La solution ou composition de n'importe laquelle des revendications précédentes, comportant de plus un lyoprotecteur, optionnellement dans laquelle le lyoprotecteur est un sucre.

13. La solution ou composition de la revendication 12, dans laquelle le lyoprotecteur est sélectionné parmi mannitol, lactose, saccharose, tréhalose et des combinaisons de ces derniers.

14. La solution ou composition de la revendication 13, dans laquelle le lyoprotecteur est tréhalose et :
(i) le tréhalose est présent en quantité allant d'environ 1 % à environ 15 % (M/V) ;
(ii) le tréhalose est présent en quantité allant d'environ 3 % à environ 9 %, par exemple environ 9 % ; ou
(iii) le ratio de protéine morphogénétique osseuse à tréhalose est d'environ 7x 10⁻⁵: 1 à environ 1,3: 1 (M/M).

15. La solution ou composition de n'importe laquelle des revendications précédentes comportant de plus un antioxydant, optionnellement sélectionné parmi méthionine, acide ascorbique, alcool benzylique, glutathion, m-crésol et thioglycérol.

16. Une composition solide qui peut être obtenue par un procédé comportant l'étape de lyophiliser la solution de n'importe laquelle des revendications 1, 2, 5, 6, 8, 9, 10, 11, 12, 13, 14 ou 15.

17. Une solution selon la revendication 1 ou la revendication 5, comportant de plus un stabilisateur sélectionné parmi proline, glycine, valine, isoleucine, et leucine, optionnellement dans laquelle le stabilisateur comporte de la proline.

18. Une composition solide comportant :
(i) une protéine morphogénétique osseuse ;
(ii) un sel de glycylglycine ou un sel de tartrate ; et
(iii) un composé sélectionné parmi proline, glycine, valine, isoleucine, leucine, un sel de proline, un sel de glycine, un sel de valine, un sel d'isoleucine, ou un sel de leucine.


### SEQUENCE LISTING

<110> Stryker Corporation Hile, David Strohmeier, Gregg
<120> BUFFERS FOR CONTROLLING THE PH OF BONE MORPHOGENETIC PROTEINS
<130> STK-096PC
<150> US 61/243,383
   <151> 2009-09-17
<160> 8
<170> PatentIn version 3.5
<210> 1
   <211> 139
   <212> PRT
   <213> Homo sapiens
<400> 1
   Ser Thr Gly Ser Lys Gln Arg Ser Gln Asn Arg Ser Lys Thr Pro Lys
   1               5                   10                  15
   Asn Gln Glu Ala Leu Arg Met Ala Asn Val Ala Glu Asn Ser Ser Ser
               20                  25                  30
   Asp Gln Arg Gln Ala Cys Lys Lys His Glu Leu Tyr Val Ser Phe Arg
           35                  40                  45
   Asp Leu Gly Trp Gln Asp Trp Ile Ile Ala Pro Glu Gly Tyr Ala Ala
       50                  55                  60
   Tyr Tyr Cys Glu Gly Glu Cys Ala Phe Pro Leu Asn Ser Tyr Met Asn
   65                  70                  75                  80
   Ala Thr Asn His Ala Ile Val Gln Thr Leu Val His Phe Ile Asn Pro
                   85                  90                  95
   Glu Thr Val Pro Lys Pro Cys Cys Ala Pro Thr Gln Leu Asn Ala Ile
               100                 105                 110
   Ser Val Leu Tyr Phe Asp Asp Ser Ser Asn Val Ile Leu Lys Lys Tyr
           115                 120                 125
   Arg Asn Met Val Val Arg Ala Cys Gly Cys His
       130                 135
<210> 2
   <211> 114
   <212> PRT
   <213> Homo sapiens
<400> 2
   Gln Ala Lys His Lys Gln Arg Lys Arg Leu Lys Ser Ser Cys Lys Arg
   1 5 10 15
   His Pro Leu Tyr Val Asp Phe Ser Asp Val Gly Trp Asn Asp Trp Ile
   20 25 30
   Val Ala Pro Pro Gly Tyr His Ala Phe Tyr Cys His Gly Glu Cys Pro
   35 40 45
   Phe Pro Leu Ala Asp His Leu Asn Ser Thr Asn His Ala Ile Val Gln
   50 55 60
   Thr Leu Val Asn Ser Val Asn Ser Lys Ile Pro Lys Ala Cys Cys Val
   65 70 75 80
   Pro Thr Glu Leu Ser Ala Ile Ser Met Leu Tyr Leu Asp Glu Asn Glu
   85 90 95
   Lys Val Val Leu Lys Asn Tyr Gln Asp Met Val Val Glu Gly Cys Gly
   100 105 110
   Cys Arg
<210> 3
   <211> 139
   <212> PRT
   <213> Homo sapiens
<400> 3
   Ser Ala Ser Ser Arg Arg Arg Gln Gln Ser Arg Asn Arg Ser Thr Gln
   1 5 10 15
   Ser Gln Asp Val Ala Arg Val Ser Ser Ala Ser Asp Tyr Asn Ser Ser
   20 25 30
   Glu Leu Lys Thr Ala Cys Arg Lys His Glu Leu Tyr Val Ser Phe Gln
   35 40 45
   Asp Leu Gly Trp Gln Asp Trp Ile Ile Ala Pro Lys Gly Tyr Ala Ala
   50 55 60
   Asn Tyr Cys Asp Gly Glu Cys Ser Phe Pro Leu Asn Ala His Met Asn
   65                  70                  75                  80
   Ala Thr Asn His Ala Ile Val Gln Thr Leu Val His Leu Met Asn Pro
                   85                  90                  95
   Glu Tyr Val Pro Lys Pro Cys Cys Ala Pro Thr Lys Leu Asn Ala Ile
               100                 105                 110
   Ser Val Leu Tyr Phe Asp Asp Asn Ser Asn Val Ile Leu Lys Lys Tyr
           115                 120                 125
   Arg Asn Met Val Val Arg Ala Cys Gly Cys His
       130                 135
<210> 4
   <211> 116
   <212> PRT
   <213> Homo sapiens
<400> 4
   Ser Pro Lys His His Ser Gln Arg Ala Arg Lys Lys Asn Lys Asn Cys
   1               5                   10                  15
   Arg Arg His Ser Leu Tyr Val Asp Phe Ser Asp Val Gly Trp Asn Asp
               20                  25                  30
   Trp Ile Val Ala Pro Pro Gly Tyr Gln Ala Phe Tyr Cys His Gly Asp
           35                  40                  45
   Cys Pro Phe Pro Leu Ala Asp His Leu Asn Ser Thr Asn His Ala Ile
       50                  55                  60
   Val Gln Thr Leu Val Asn Ser Val Asn Ser Ser Ile Pro Lys Ala Cys
   65                  70                  75                  80
   Cys Val Pro Thr Glu Leu Ser Ala Ile Ser Met Leu Tyr Leu Asp Glu
                   85                  90                  95
   Tyr Asp Lys Val Val Leu Lys Asn Tyr Gln Glu Met Val Val Glu Gly
               100                 105                 110
   Cys Gly Cys Arg
           115
<210> 5
   <211> 132
   <212> PRT
   <213> Homo sapiens
<400> 5
   Asn Gln Asn Arg Asn Lys Ser Ser Ser His Gln Asp Ser Ser Arg Met
   1               5                   10                  15
   Ser Ser Val Gly Asp Tyr Asn Thr Ser Glu Gln Lys Gln Ala Cys Lys
               20                  25                  30
   Lys His Glu Leu Tyr Val Ser Phe Arg Asp Leu Gly Trp Gln Asp Trp
           35                  40                  45
   Ile Ile Ala Pro Glu Gly Tyr Ala Ala Phe Tyr Cys Asp Gly Glu Cys
       50                  55                  60
   Ser Phe Pro Leu Asn Ala His Met Asn Ala Thr Asn His Ala Ile Val
   65                  70                  75                  80
   Gln Thr Leu Val His Leu Met Phe Pro Asp His Val Pro Lys Pro Cys
                   85                  90                  95
   Cys Ala Pro Thr Lys Leu Asn Ala Ile Ser Val Leu Tyr Phe Asp Asp
               100                 105                 110
   Ser Ser Asn Val Ile Leu Lys Lys Tyr Arg Asn Met Val Val Arg Ser
           115                 120                 125
   Cys Gly Cys His
       130
<210> 6
   <211> 120
   <212> PRT
   <213> Homo sapiens
<400> 6
   Ala Pro Leu Ala Thr Arg Gln Gly Lys Arg Pro Ser Lys Asn Leu Lys
   1               5                   10                  15
   Ala Arg Cys Ser Arg Lys Ala Leu His Val Asn Phe Lys Asp Met Gly
               20                  25                  30
   Trp Asp Asp Trp Ile Ile Ala Pro Leu Glu Tyr Glu Ala Phe His Cys
           35                  40                  45
   Glu Gly Leu Cys Glu Phe Pro Leu Arg Ser His Leu Glu Pro Thr Asn
       50                  55                  60
   His Ala Val Ile Gln Thr Leu Met Asn Ser Met Asp Pro Glu Ser Thr
   65                  70                  75                  80
   Pro Pro Thr Cys Cys Val Pro Thr Arg Leu Ser Pro Ile Ser Ile Leu
                   85                  90                  95
   Phe Ile Asp Ser Ala Asn Asn Val Val Tyr Lys Gln Tyr Glu Asp Met
               100                 105                 110
   Val Val Glu Ser Cys Gly Cys Arg
           115                 120
<210> 7
   <211> 120
   <212> PRT
   <213> Homo sapiens
<400> 7
   Thr Ala Phe Ala Ser Arg His Gly Lys Arg His Gly Lys Lys Ser Arg
   1               5                   10                  15
   Leu Arg Cys Ser Lys Lys Pro Leu His Val Asn Phe Lys Glu Leu Gly
               20                  25                  30
   Trp Asp Asp Trp Ile Ile Ala Pro Leu Glu Tyr Glu Ala Tyr His Cys
           35                  40                  45
   Glu Gly Val Cys Asp Phe Pro Leu Arg Ser His Leu Glu Pro Thr Asn
       50                  55                  60
   His Ala Ile Ile Gln Thr Leu Met Asn Ser Met Asp Pro Gly Ser Thr
   65                  70                  75                  80
   Pro Pro Ser Cys Cys Val Pro Thr Lys Leu Thr Pro Ile Ser Ile Leu
                   85                  90                  95
   Tyr Ile Asp Ala Gly Asn Asn Val Val Tyr Lys Gln Tyr Glu Asp Met
               100                 105                 110
   Val Val Glu Ser Cys Gly Cys Arg
           115                 120
<210> 8
   <211> 129
   <212> PRT
   <213> Homo sapiens
<400> 8
   Thr Ala Leu Ala Gly Thr Arg Thr Ser Gln Gly Ser Gly Gly Gly Ala
   1               5                   10                  15
   Gly Arg Gly His Gly Arg Arg Gly Arg Ser Arg Cys Ser Arg Lys Pro
               20                  25                  30
   Leu His Val Asp Phe Lys Glu Leu Gly Trp Asp Asp Trp Ile Ile Ala
           35                  40                  45
   Pro Leu Asp Tyr Glu Ala Tyr His Cys Glu Gly Leu Cys Asp Phe Pro
       50                  55                  60
   Leu Arg Ser His Leu Glu Pro Thr Asn His Ala Ile Ile Gln Thr Leu
   65                  70                  75                  80
   Leu Asn Ser Met Ala Pro Asp Ala Ala Pro Ala Ser Cys Cys Val Pro
                   85                  90                  95
   Ala Arg Leu Ser Pro Ile Ser Ile Leu Tyr Ile Asp Ala Ala Asn Asn
               100                 105                 110
   Val Val Tyr Lys Gln Tyr Glu Asp Met Val Val Glu Ala Cys Gly Cys
           115                 120                 125
   Arg